# EUROPEAN PATENT APPLICATION

(11) **EP 2 664 353 A1**
(43) Date of publication of application: **20.11.2013**
(21) Application number: 13168256.9
(22) Date of filing: 17.04.2009
(51) Int. Cl.: A61M 25/00, A61M 25/06, A61M 25/01

(54) **A method for positioning an access sheath and a security guide**

(30) Priority: 18.04.2008 FR 0802180; 24.06.2008 US 144688
(62) Divisional of application: 09733279.5
(71) Applicant: Coloplast A/S, 3050 Humlebaek (DK)
(72) Inventor: Lafitte, Mathieu, 24200 Sainte Nathalene (FR); Callede, David, 24200 Sarlat la Caneda (FR); Charpiot, Patrick, 92290 Chatenay Malabry (FR)

(57) **Abstract**

The invention relates to a method for positioning an access sheath and a security guide next to the sheath for an intervention in a difficult-to-access area within the body using only one guide.

## Description

The field of the invention relates to catheters and access sheaths through which surgeons can make an intervention with a tool in a difficult-to-access area within the human body. They are used, among others, in urology. Furthermore, that is in this domain that the problem that has given rise to the invention of the present application has arisen. However, the Applicant does not intend to limit the scope of their rights to this particular application.

When a surgeon wants to access to a kidney, if the surgical intervention is dismissed, he has, starting from the natural routes of entry, to go up inside the urethra, pass the bladder and then, beyond the ureteral meatus, go up inside the ureter to reach the kidney.

Up to now, the positioning of an access sheath was performed by a quite restrictive method.

With the aid of a cystoscope, which is a tubular element carrying an optical fiber - it is a bladder endoscope -, previously introduced inside the bladder, a first radiopaque guide is driven up to the bladder. Then, with the aid of the cystoscope, the ureteral meatus is targeted at in order to introduce the guide inside the ureter. The guide is a generally sheathed nickel, titanium and/or stainless steel alloy lead.

After the setting up of this first guide, a radiopaque double channel ureteral probe is engaged by one of its both channels onto the guide and it is driven up to the ureter. Through the other channel of the ureteral probe, a second guide is introduced till the ureter. Then the ureteral probe is removed to let behind only both guides, a working guide and a security guide, this latter being fixed onto the patient.

During these first steps, the radiopaque components have been visualized to check their positions.

The access sheath being threaded into a dilator projecting forwards outside the sheath, the access sheath is engaged into the working guide through the dilator channel, then the sheath end is driven up to a position between the bladder and the kidney, but nearer the bladder. The dilator and the working guide are removed to let behind in place only the access sheath, and near it, the security guide that can be used in case of difficulty.

The course of all these steps of positioning the access sheath highlights the fundamental problem of the invention: reducing the number of positioning steps in order to save time and reduce the risks for the patient and limiting the number of necessary components (working and security guides, ureteral probe for positioning the security guide and the dilator).

Thus, the invention of the present application relates first to a method for positioning an access sheath and a security guide next to the sheath, for an intervention in a difficult-to-access area of the body, comprising the steps of
- engaging, driving up and setting up an ureteral probe onto the guide, **characterized in that**
- the access sheath being mounted onto the ureteral probe also plays a role of dilator, after setting up of the probe on the guide,
- the access sheath is driven up onto the probe in order to set it up, have the guide exit from the probe and lay it next to the sheath and
- the ureteral probe-dilator is removed.

According to this method, only one guide has been used, it was started to be used as a working guide before being turned into a security guide. The reduction of the number of components is obvious as well as the number of placing steps thanks to the double function of the ureteral probe i) for positioning the security guide and ii) as a dilator.

Preferably, the ureteral probe comprising a setting up channel and a channel for placing a security guide, for setting up the probe, it is engaged by the inner end of its security channel onto the guide before driving up the access sheath onto the probe.

The inner end of the channel means its end intended, after setting up, to be in an area of the intervention or in the nearest from this area. Conversely, the inner end of the channel is that which will be at the entry of the natural route of the body or the nearest one from this natural entry.

The positioning method of the invention can also resort to two different guides, a working guide and a security guide.

The ureteral probe comprising a setting up channel and a channel for positioning a security guide, for setting up the probe, it is engaged through the inner end of its setting up channel on the working guide, then, after setting up, a security guide is introduced inside the security channel of the probe through its external end, before pulling up the access sheath on the probe to have the security guide exit from the probe and extending it next to the sheath and then removing the probe and the working guide.

This two guide positioning method should be better suitable to less experimented operators.

The invention also relates to a catheter for positioning an access sheath and a security guide next to the sheath for an intervention in a difficult-to-access area within the body, comprising, along the catheter, at least one security channel for positioning a guide, characterized by the fact that the security channel is a channel extending between an outer hole and an inner hole, the wall of the catheter extending between both holes of the security channel being arranged to open under the action of a peel strength directed from inside to outside the channel.

Thus, when this catheter is used as a ureteral probe onto which an access sheath is threaded and that a guide extends inside the security channel, whether it is a security guide or a working guide to be turned into a security guide, when the access sheath is driven up on the catheter, the inner end of the sheath which is arranged between the wall of the catheter and the guide exerts on the guide a peeling or stripping action, which is well directed from inside to outside of the channel since the guide is located inside the channel, such action having the effect to open the wall of the catheter and make the guide exit to free it.

It is meant by outer hole, the second port, inside the wall of the catheter, through which the guide is to exit; it can be a hole arranged to this end and so the arrangement of the catheter allowing action of peeling extends between these two holes.

But the wall of the catheter can also be arranged in such a way along its whole length and so the outer hole is only the second outlet port of the guide, after a previous peeling action. In this area of the second outlet port of the guide, ramp means may have been arranged to have the guide exit more easily.

The security channel can be split between both inner and outer holes, the lips of the split being joined or mutually overlying at rest state.

The wall of the security channel can also comprise a breaking line between both inner and outer holes and thus present the quality of being splittable.

The inner hole of the security channel can be situated at the inner end of the catheter or in this side of it.

When the catheter does only comprise the security channel, the channel is said of security since it serves for positioning a security guide. But during the setting up, it mainly serves as a working channel.

The security channel can extend along the whole length of the catheter, but with an intermediate outlet port.

The catheter can also comprise a second working channel completely extending along the catheter between its both inner (distal) and outer (proximal) ends.

Thus, the working channel can receive a working guide and the security channel, a security guide.

The invention also relates to an assembly of the catheter according to the invention and an access sheath threaded onto the catheter.

It is to be noticed that U.S. Patent n° 2007/0,149,948 does not disclose a catheter adapted for positioning an access sheath, but a proper access sheath with a working channel comprising a guiding thread for substituting the sheath for another one.

The invention of the present application relies on the characteristic of the working channel with a sheath weakened area of said prior art, but for its use on a safety channel of a sheath positioning catheter. Applying a weakened area for a sheath positioning catheter and not for the sheath itself is an approach method perfectly worth protecting.

The invention will be better understood with the help of the following description of an interesting embodiment of the catheter-access sheath assembly and of the positioning method of the invention, with reference to the attached drawing wherein:

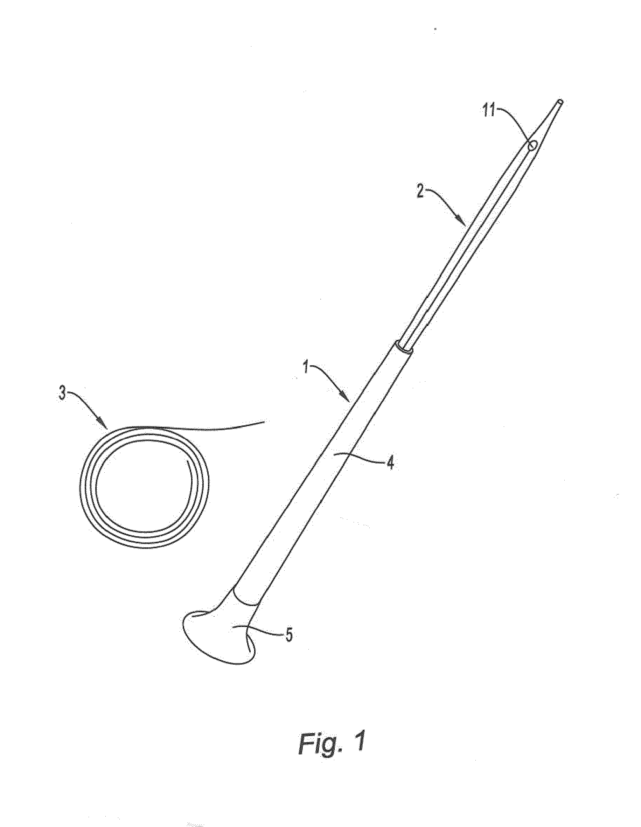

- Fig. 1 is a perspective view of the assembly of the catheter and the access sheath arranged on it, with the guide;

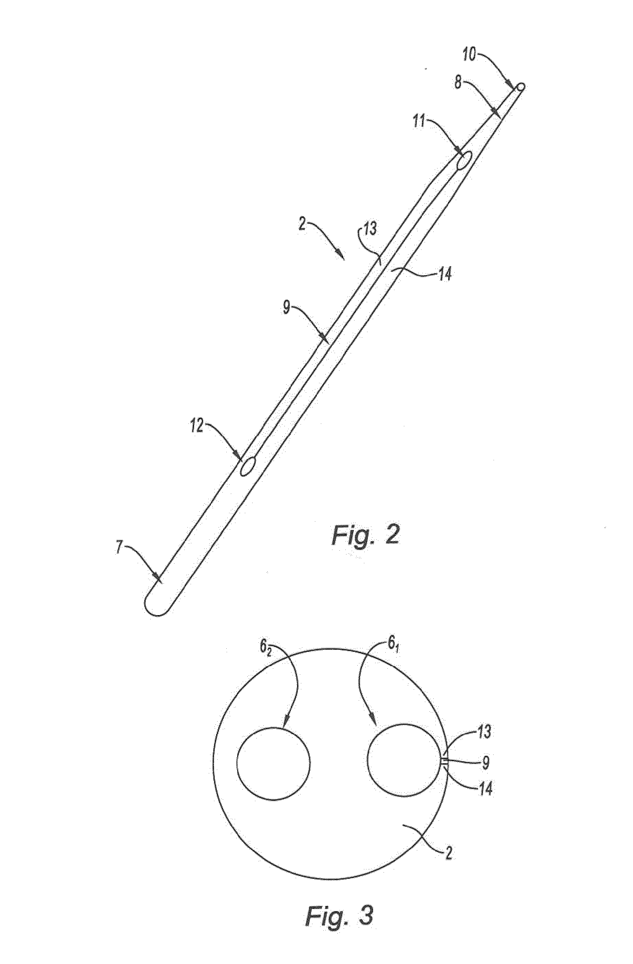

- Fig. 2 is a perspective view of the catheter;
- Fig. 3 is cross-sectional view of the catheter of Fig. 1 and 2;
- Fig. 4 is a perspective view of the assembly of the catheter and the access sheath positioned on the guide;
- Fig. 5 is a perspective view of the assembly of Fig. 4, the access sheath being driven up onto the catheter and the released guide, extending next to the sheath.

The access sheath 1, the positioning method of which will be now described, is a flexible tube but collapse resistant, for access to the urinary tract. The sheath 1 is slid onto a catheter, here a ureteral probe 2 for receiving one or more guides 3.

The sheath 1 comprises a reinforced tube 4 to resist to compression/plication, and a bucket 5 on its proximal end, to allow for an easy introduction of instruments/tools.

For the positioning thereof, the access sheath 1 has been previously arranged on a ureteral probe 2 adapted to serve as a dilator.

The ureteral probe 2 is a tube comprising two separate longitudinal hollow channels 6₁ and 6₂ and both extending here between a proximal end 7 and a distal end 8 conformed to facilitate its insertion. The end 8 is here lightly frustoconical tapering to the tip 10. One (6₁) of both channels is here partly split (9) between two outlet holes, or ports, 11, 12 of the channel 61 arranged in the wall of the probe, the inner hole 11, in the tapered end portion of the probe 2, near the distal end 8, and the outer hole 12, between the middle of the length of the probe 2 and the proximal end 7. An outlet hole is also called an eye. Along the slit channel 61, there thus extends a split 9 between two lips 13, 14 which, at rest, are joined.

The split 9 can extend along the whole length of the probe, with or without outer eye; in absence of an eye, a guide would exit from the slit channel 61 of the probe 2, after peeling, in an area of the wall called outlet port and corresponding to the eye 12.

At rest, the lips 13, 14 of the split 9 are joined.

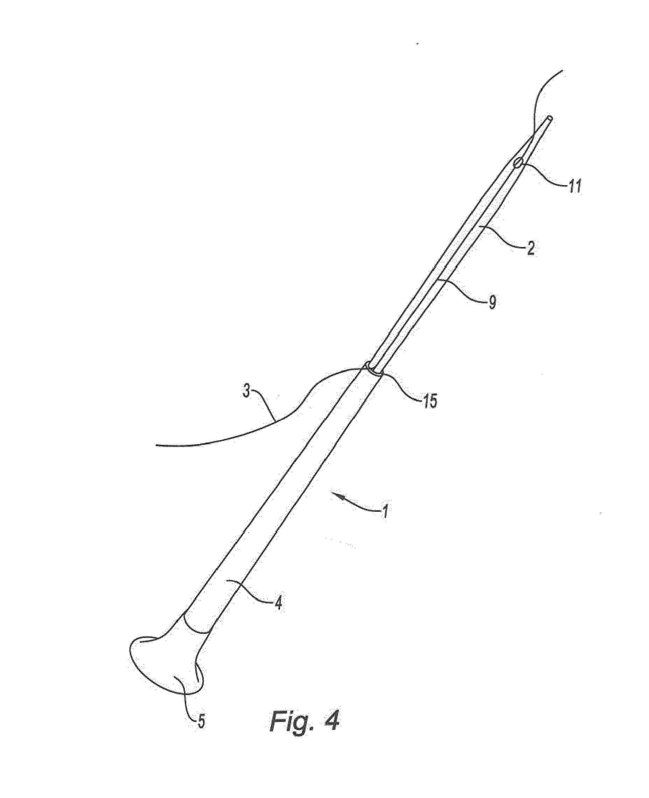

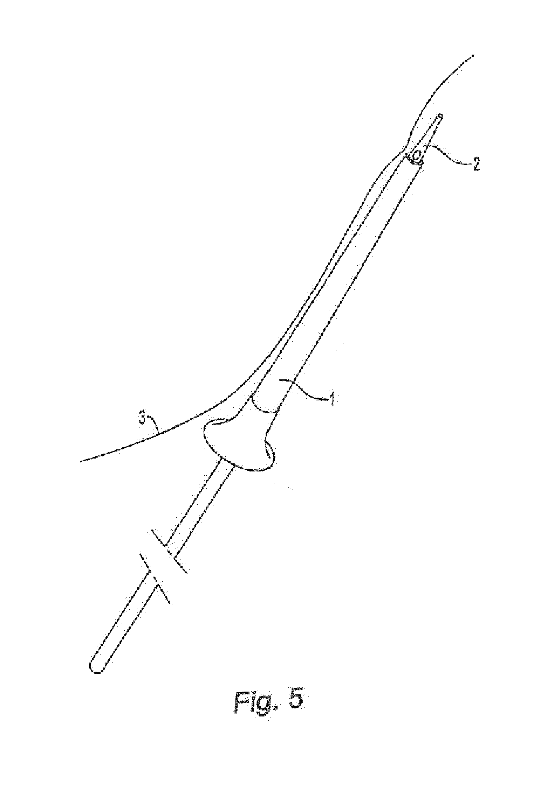

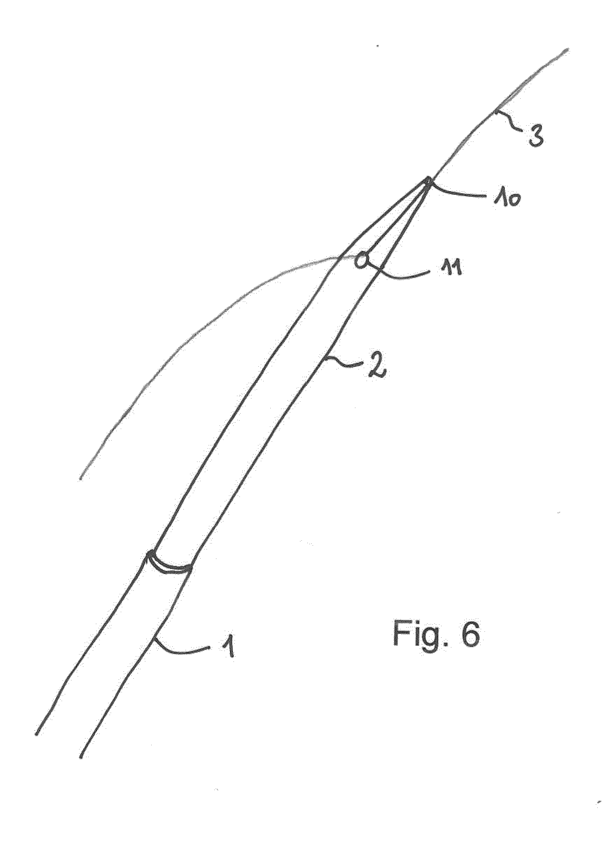

The access sheath 4 on the ureteral probe 2 has been described, comprising two channels 6₁ and 6₂ and their positioning will now be described with the only guide 3 which will start to serve as a working guide (Fig. 4), before being turned into a security guide (Fig. 5).

The guide 3 will be first positioned up to the ureter, for example, using a cystoscope, preliminarily introduced into the bladder. The guide 3 being positioned, after introduction through the natural entry route, up to the ureter, the probe 2 is engaged onto the guide 3 through its inner hole 11 of the split channel 61, near the distal end 8. By further driving up the probe 2 along the guide 3, the end of the guide 3 exits again from the outer hole 12, at the middle of the probe. Subsequently the sheath 1 is driven up on the probe 2, which leads to the release of the guide 3 away from the probe 2 (Fig. 5), the guide 3 being positioned and extending itself next to the sheath 1. The ureteral probe-dilator 2 is then removed.

Moving the sheath 1 up on the probe 2, the front edge 15 of the sheath pushes and pulls the guide 3 outside the split 9 of the slit channel of the probe 2, such a split opening through spacing apart the lips 13, 14 thereof under the action of such tearing force or peeling force, directed from inside to outside the channel.

### Embodiments

1. Method for positioning an access sheath (1) and a security guide (3) next to the sheath, for an intervention in a difficult-to-access area within the body, comprising the steps of:
   - positioning a guide (3) and
   - engaging, driving up and setting up an ureteral probe onto the guide,
      **characterized in that**
   - the access sheath (1) being mounted on the ureteral probe (2) also plays a role of dilator, after setting up of the probe (2) on the guide (3),
   - the access sheath (1) is driven up onto the probe (2) in order to set it up, have the guide (3) exit from the probe (2) and lay it next to the sheath (1) and
   - the ureteral probe-dilator (2) is removed.
2. Method according to embodiment 1, wherein the ureteral probe (2) comprising a setting up channel and a channel (6₁) for positioning a security guide (3), for setting up the probe (2), is engaged through the inner end (11) of its security channel (6₁) onto the guide (3) before driving up the access sheath (1) onto the probe (2).
3. Method according to embodiment 1, wherein the ureteral probe (2) comprising a setting up channel (6₂) and a channel (6₁) for positioning a security guide (3), for setting up the probe (2), is engaged through the inner end (10) of its setting up channel (6₂) onto the working guide, then, after setting up, a security guide (3) is introduced inside the security channel (6₁) of the probe (2) through its outer end (7, 12) before driving up the access sheath (4) of the probe (2) to have the probe (2) exit from the security guide (3) and lay it next to the sheath (1) and then remove the probe and the working guide.
4. Catheter (2) for positioning an access sheath (1) and a security guide (3) next to the sheath (1) for an intervention in a difficult-to-access area within the body, comprising, along the catheter (2), at least one security channel (61) for positioning a guide (3), characterized by the fact that the security channel (6₁) is a channel extending between an outer hole (12) and an inner hole (11), the wall of the catheter extending between both holes of the security channel being arranged to open under the action of a peel strength directed from inside to outside the channel.
5. Catheter according to embodiment 4, wherein the outer hole (12) is a outlet guide hole (3) arranged to this end.
6. Catheter according to embodiment 4, wherein the wall of the catheter (2) is arranged to open on its whole length and the outer hole is the second outlet port of the guide (3).
7. Catheter according to embodiment 4, wherein the outer hole is an outlet port of the guide comprising ramp means arranged for the outlet of the guide.
8. Catheter according to any of embodiments 4 to 7, wherein the security channel (6₁) is split (9) between both inner (11) and outer (12) holes, the lips of the split being joined at rest state.
9. Catheter according to any of embodiments 4 to 7, wherein the wall of the security channel (61) comprises a breaking line between both inner (11) and outer (12) holes.
10. Catheter according to any of embodiments 4 to 9, wherein the inner hole (11) of the security channel (61) is located at the inner end (8) of the catheter.
11. Catheter according to embodiment 4, wherein the security channel extends along the whole length of the catheter (2).
12. Catheter according to embodiment 4, wherein a second working channel (6₂) is provided, completely extending along the catheter between its both distal (10) and proximal (7) ends.
13. Assembly of the catheter according to any of embodiments 4 to 12 and the access sheath (1) threaded onto the catheter (2).

## Claims

1. Method for positioning an access sheath (1) and a security guide next to the sheath, for an intervention in a difficult-to-access area within the body, comprising the steps of:
- engaging, driving up and setting up an ureteral probe (2) onto a guide (3),
**characterized in that**
- the access sheath (1) being mounted on the ureteral probe (2) also plays a role of dilator, after setting up of the ureteral probe (2) on the guide (3),
- the access sheath (1) is driven up onto the ureteral probe (2) in order to set it up, have the guide (3) exit from the ureteral probe (2) and lay it next to the sheath (1) and
- the ureteral probe (2) is removed.

2. Method according to claim 1, wherein the ureteral probe (2) comprises a setting up channel and a security channel (6₁) for positioning the guide (3) wherein, for setting up the ureteral probe (2), it is engaged through the inner end (11) of its security channel (6₁) onto the guide (3) before driving up the access sheath (1) onto the ureteral probe (2).

3. Method according to claim 1, wherein the ureteral probe (2) comprises a setting up channel (6₂) and a security channel (6₁) for positioning a security guide (3) wherein, for setting up the ureteral probe (2), it is engaged through the inner end (10) of its setting up channel (6₂) onto a working guide, then, after setting up, a security guide is introduced inside the security channel (6₁) of the ureteral probe (2) through its outer end (7, 12) before driving up the access sheath (1) on the ureteral probe (2) to have the ureteral probe (2) exit from the security guide and lay it next to the access sheath (1) and then remove the ureteral probe (2) and the working guide.

4. Method according to any one of the preceding claims further comprising an initial step of positioning a guide (3).

5. Method according to any one of the preceding claims wherein said guide (3) is used as working guide before being turned into a security guide.
